Europäisches Patentamt

European Patent Office

Office européen des brevets

(11) Publication number: **0 076 676**
**B1**

(19)

(12) EUROPEAN PATENT SPECIFICATION

(45) Date of publication of patent specification: **30.12.86**

(51) Int. Cl.⁴: **A 61 K 37/02, C 07 K 7/44**

(21) Application number: **82305254.3**

(22) Date of filing: **04.10.82**

(54) **Process for using endorphins as antitumour agents.**

(30) Priority: **05.10.81 US 308287**

(43) Date of publication of application:
**13.04.83 Bulletin 83/15**

(45) Publication of the grant of the patent:
**30.12.86 Bulletin 86/52**

(84) Designated Contracting States:
**BE DE FR GB IT**

(56) References cited:
**none**

(73) Proprietor: **Plotnikoff, Nicholas P.**
**7777 South Lewis**
**Tulsa Oklahoma, 74136 (US)**

(72) Inventor: **Plotnikoff, Nicholas P.**
**7777 South Lewis**
**Tulsa Oklahoma, 74136 (US)**

(74) Representative: **Thomson, Paul Anthony et al**
**Potts, Kerr & Co. 15, Hamilton Square**
**Birkenhead Merseyside L41 6BR (GB)**

**The file contains technical information
submitted after the application was filed and
not included in this specification**

Courier Press, Leamington Spa, England.

EP 0 076 676 B1

# 0 076 676

**Description**

This invention relates to the use of an endogenous endorphin for the manufacture of a medicament for inhibiting tumorous growth and/or stimulating the natural immune system resisting tumorous growth. More specifically this invention relates to the use of leucine-enkephalin and methionine-enkephalin.

Since the discovery, isolation and the complete analysis of the amino acid sequence of specific endogenous, highly reactive and incredibly powerful peptides (e.g. interferon, endorphins and enkephalins), a rebirth and revitalization of interest and activity in biochemical research has taken place. During the last decade the industrial world's race to be the first to commercially synthesize interferon is not only a daily topic on Wall Street but is also well known to the layman and casual observer. Perhaps as at no other time is history has the phrase "the miracles of modern medicine" been more applicable as a genuine expectation.

Consistent with the contemporary so-called 'lock and key' theory, the existence of specific receptors in the brain for morphine-like substances has been established and corresponding endogenous specific ligands have been located and identified to be two pentapeptides, methionine-enkephalin and leucine-enkephalin (Hughes et al, Nature, *258* 577—579 (1975); Simantov et al, Proc. Natl. Acad. Sci., U.S.A., *73* 2515—2519 (1976); Kosterlitz, *Opiates and Endogenous Opioid Peptides*, Elsevier/North Holland Biomedical Press (1976)). Other larger peptides, the endorphins, have also been found to bind to morphine receptors (Li et al, Nature *260* 622—24 (1976); Cox et al, Proc. Natl. Acad. Sci., U.S.A., *73* 1821—23 (1976): Segal et al, Science *198* 411—413 (1977)). Subsequent studies elaborated on the analgesic activity in various animal models by the intracerebral route of administration. However, there appeared to be lack of analgesic activity when the peptides were administered by the intravenous (or intramuscular or intraperitoneal) routes of administration. This dichotomy of analgesic activity by different routes of administration (brain versus peripheral) was explained in terms of blood-brain barrier differences as well as a rapid rate of metabolism in plasma.

However, pronounced activity by systemic administration was discovered by Plotnikoff et al (Life Sci. *19* 1283—1288 (1976)), who showed that the enkephalins-endorphins exhibited marked activity as tranquilizers and antidepressants. Most important, Plotnikoff demonstrated that the enkephalins were extremely active in potentiating the central effects of dopamine.

In 1979, Wybran et al reported that normal human blood T lymphocytes bear surface receptor-like structures for methionine-enkephalin and recent discoveries further support the view that T-cell lymphocytes were covered with enkephalin and endorphin receptor sites.

In the same year, the New York Academy of Sciences Symposium on Subcellular Factors in Immunity (Volume 332, Dec. 28, 1979) disclosed that the thymus gland of animals and humans was secreting peptides (thymosin and others) that controlled activities of T-cell lymphocytes and associated cells. These thymus peptides were found to have anti-neoplastic effects in animals and humans. In addition, the thymus peptides were found to control the ageing process. Finally, the thymus peptides appear to regulate 'auto-immune responses' of lymphocyte particles.

In view of the above, a process has been discovered for inhibiting tumorous growth and stimulating the natural immune system resisting tumorous growth comprising the step of treating the tumorous growth, *in vivo*, with an endogenous endorphin, including beta-endorphin (61—91) and corresponding peptides derived from beta-endorphin (61—91) as the precursor. It is further provided that the endogenous endorphin be leucine-enkephalin or methionine-enkephalin and that they be administered at from 0.001 to 30 mg/kg.

An object of the present invention is the use of endogenous endorphin for the manufacture of a medicament for promoting and stimulating the immune system's resistance to neoplastic cellular growth. A further object is the use for the same purpose of leucine-enkephalin and methionine-enkephalin.

Starting with the two facts: 1) T-cell lymphocytes are covered with enkephalin and endorphin receptor sites and 2) the thymus gland of animals and humans secretes peptides (thymosin and others) that control the activities of T-cell lymphocytes and associated cells, a working hypothesis is proposed that the hypothalamus secretes a releasing factor and also a releasing inhibitory factor to the pituitary gland (storehouse of beta-endorphin). Therefore, the beta-endorphins' release and inhibition of release are controlled by factors from the hypothalamus. The release of beta-endorphin from the pituitary results in stimulation of the adrenals and thymus and/or T-cell lymphocytes and the entire immune cascade of responses. Beta-endorphin is probably metabolized in the bloodstream into its numerous fragments (alpha- and gamma- endorphin as well as the smaller enkephalins). These smaller fragments are active in stimulating the immune systems.

In order to test the hypothesis and to establish the efficacy of these smaller fragments, the following series of *in vivo* experiments was designed and performed, using laboratory mice inoculated with tumorous cells and then treated with leucine-enkephalin and methionine-enkephalin. Groups of $BDF_1$ female mouse strain (16—20 gms) were inoculated with L1210 (leukemia) tumor cells ($1 \times 10^4$, $1 \times 10^3$, or $1 \times 10^2$ cells) and observed for survival. The L1210 line of tumor cells was maintained in DBA/2 host mice. Ascitic fluid from the DBA/2 mice was used to inoculate the $BDF_1$ mice.

2

Example I

Three separate experiments were conducted utilizing ten mice per group. The first experiment involved inoculating all mice with $1\times10^4$ cells, the second with $1\times10^3$ cells, and the third with $1\times10^2$ cells. Control groups were administered saline subcutaneously daily, while the methionine-enkephalin groups and the leucine-enkephalin groups were administered 10 mg/kg s.c. daily. The survival rate of the mice was monitored and the corresponding data for the three experiments are presented in tables I, II, and III respectively.

TABLE I

$1\times10^4$ L1210 Tumor cells in $BDF_1$ mice—effects of enkephalins

| Days | Methionine-enkephalin 10 mg/kg s.c.* | Controls | Leucine-enkephalin 10 mg/kg s.c.* |
|------|--------------------------------------|----------|------------------------------------|
| 11 | 6 died | 5 died | 8 died |
| 12 | 1 died | 2 died | 0 died |
| 13 | 2 died | 3 died | 2 died |
| | $\frac{9}{10}$ died | $\frac{10}{10}$ died | $\frac{10}{10}$ died |

*Enkephalin injections started on day 2

TABLE II

$1\times10^3$ L1210 Tumor cells in $BDF_1$ Mice-effects of enkephalins

| Days | Methionine-enkephalin 10 mg/kg s.c. | Controls | Leucine-enkephalin 10 mg/kg s.c. |
|------|-------------------------------------|----------|-----------------------------------|
| 13 | 0 died | 2 died | 2 died |
| 14 | 4 died | 2 died | 4 died |
| 15 | 3 died | 5 died | 0 died |
| 16 | 1 died | 1 died | 3 died |
| 17 | 1 died | 0 died | 0 died |
| 18 | 1 died | 0 died | 1 died |
| | $\frac{10}{10}$ died | $\frac{10}{10}$ died | $\frac{10}{10}$ died |

3

TABLE III

$1\times10^2$ L1210 Tumor cells in $BDF_1$ mice-effects of enkephalins

| Days* | Methionine-enkephalin 10 mg/kg s.c. | Controls | Leucine-enkephalin 10 mg/kg s.c. |
|---|---|---|---|
| 15 | 3 died | 5 died | 1 died |
| 16 | 2 died | 1 died | 1 died |
| 18 | 0 died | 1 died | 1 died |
| 19 | 1 died | 0 died | 0 died |
| 20 | 1 died | 1 died | 0 died |
| 21 | 0 died | 0 died | 0 died** |
| | 9 died | 9 died | 3 died |

*Enkephalin injections started on day two
**Chi square test $p<0.025$ between controls and leucine-enkephalin,

With the high tumor cell count of $1\times10^4$, no differences in survival times between controls, methionine-enkephalin, or leucine-enkephalin treated groups were seen (Table I). In the experiment in which the mice were inoculated with $1\times10^3$ tumor cells (Table II), survival time for the controls was 16 days, 18 days for the methionine-enkephalin group, and 18 days for the leucine-enkephalin treated group. However, in the third experiment, carried out at $1\times10^2$ tumor cells, the survival time for the controls as well as the methionine-enkephalin treated group was 20 days, while the leucine-enkephalin group had a survival time of >20 days.

The seven surviving mice from experiment three were continued on daily leucine-enkephalin medication (10 mg/kg i.p.) until day 36. The mice did not receive any additional leucine-enkephalin medication on subsequent days of the experiment. On day 37, the surviving seven mice were rechallenged with an additional inoculum of tumor cells ($1\times10^4$ cells). One of the seven mice so treated died on day 52.

The remaining six mice survived until day 64, at which time they were rechallenged further with a higher inoculum of tumor cells ($1\times10^6$). All six miced died by day 70. These rechallenge studies suggest that leucine-enkephalin stimulates the immune system as measured by survival of the test animals.

Example II

A second set of three experiments was performed in a manner similar to the experiments of Example I except that 25 mice were used as controls, 30 mice were treated with methionine-enkephalin and another 30 were treated with leucine-enkephalin. In each case the mice were inoculated with $1\times10^4$ tumor cells and the enkephalin was administered daily at 30 mg/kg s.c. The data related to the survival of the mice are presented in Table IV.

TABLE IV

$1 \times 10^4$ L1210 Tumor cells in BDF$_1$ mice-effects of enkephalins

| Days | Controls | 30 mg/kg Methionine-enkephalin | 30 mg/kg Leucine-enkephalin |
|---|---|---|---|
| | | # dead/# tested | |
| 13 | 7/25 | 5/30 | 3/30 |
| 14 | 10/25 | 7/30 | 5/30 |
| 15 | 16/25 | 8/30 | 12/30 |
| 16 | 20/25 | 12/30 | 17/30 |
| 17 | 21/25 | 15/30 | 22/30 |
| 18 | 21/25 | 18/30 | 22/30 |
| 19 | 21/25 | 18/30 | 26/30 |
| 20 | 22/25 | 19/30 | 26/30 |
| 21 | 22/25 | 19/30 | 26/30 |

As indicated in Table IV, approximately half the control mice died by the fourteenth day. In contrast, half of the methionine-enkephalin treated mice died by the seventeenth day. By day 21 of the experiment, eleven out of the thirty treated mice had survived.

Delayed deaths were also seen in mice treated with leucine-enkephalin. The median day of death was about sixteen days. Thus, the last three experiments (Example II) demonstrate that the higher doses of both leucine- and methionine- enkephalin (30 mg/kg) are effective in prolonging survival of mice inoculated with $1 \times 10^4$ cells. Of great interest is the apparent potency of methionine-enkephalin, resulting in survival at day 21 of 11 of 30 mice. This survival finding may be related to and be consistent with the previous observation that specific receptor binding sites for the enkephalins are present on T lympocytes.

A dramatic increase in survival time of the leucine-enkephalin treated mice in the third experiment of Example I, and the sharp contrast of these leucine-enkephalin treated mice when compared both to the control mice and to the methionine-enkephalin treated mice (9 out of 10 expired between days 14—20), can be seen when comparing the results of the third experiment to those of the first and second experiments of Example I (Tables I—III). This indicates that there is remarkable and sharp separation in the activities of leucine-versus methionine- enkephalin at lower inoculation relates ($1 \times 10^2$ cells) and these effects persist even after rechallenging with tumor cells. This is again consistent with the original hypothesis and also suggests that leucine-enkephalin plays a neuroendocrine messenger role between the central nervous system and the immune system.

Since Blalock et al, Proc. Natl. Acad. Sci. *77* 10, 5972 (1980) reported that "antigenic and structural similarities among leukocyte interferon, ACTH, and gamma-endorphin imply leukocyte interferon may be a precursor or is derived from a common precursor to these hormones". Furthermore, Laidow et al observed that both methionine-enkephalin and leucine-enkephalin as well as beta-endorphin inhibit phenylalanyl-t RNA synthetase, J.B.C. *255* 4, 11908 (1980). The anti-tumor mechanisms of action of the enkephalins may be mediated through the immune systems by 1) T lymphocyte surface receptors, 2) interferon, and 3) inhibition of phenylalanyl-t RNA synthetase. And more important, the same logic leads one to conclude that the observed significant protective effects of methionine-enkephalin and the anti-tumor activity of leucine-enkephalin of the present invention would be expected from their corresponding "precursor" molecules and molecules of similar structure derived from a common precursor. Further, support for this statement can be found in and is implicit in the very presence of literature references indicating that "precursor" molecules bind to receptor sites of leukocytes (lymphocytes) : Hazum et al, Science, 205, 7, 1033—1035 (1980). Thus for purposes of this invention the following closely related enkephalins and endorphins are to be considered equivalents:

5

TABLE V
Peptide structures

| | |
|---|---|
| Met-enkephalin | Tyr-Gly-Gly-Phe-Met |
| Leu-enkephalin | Tyr-Gly-Gly-Phe-Leu |
| (Arg$^6$)-Leu-enkephalin | Tyr-Gly-Gly-Phe-Leu-Arg |
| Alpha-Neo-endorphin | Tyr-Gly-Gly-Phe-Leu-Arg-Lys-Tyr-Pro-Lys |
| Beta-Neo-endorphin | Tyr-Gly-Gly-Phe-Leu-Arg-Lys-Tyr-Pro |
| Dynorphin | Tyr-Gly-Gly-Phe-Leu-Arg-Arg-Ile-Arg-Pro |
| PH-8P (Dynorphin (1—8)) | Tyr-Gly-Gly-Phe-Leu-Arg-Arg-Ile |
| Beta-endorphin | (61—91) |
| Alpha-endorphin | |
| Gamma-endorphin | |

The effective amount of endogenous peptide to be administered will vary depending on the circumstances and end result desired. Preferably, the dose ranges that can be used therapeutically are from about 0.001 to about 30 mg/kg. The method of treatment and administration can be by any of the known routes including but not limited to: oral, i.v., i.m., s.c., aerosol, nasal, eyes, or vaginal and suppositories. The enkephalins and endorphins can be used singly or in combination, or in combination with other known chemotherapeutic agents for the treatment of all forms of neoplastic activity or related conditions.

**Claims**

1. Use of an endogenous endorphin for the manufacture of a medicament for inhibiting tumorous growth and/or stimulating the natural immune system resisting tumorous growth.

2. Use of an endogenous endorphin according to claim 1, characterized in that the endogenous endorphin is selected from beta-endorphin (61—91) and peptides of which beta-endorphin (61—91) is the precursor.

3. Use of an endogenous endorphin according to claim 1, characterized in that the endogenous endorphin is selected from met-enkephalin, leu-enkephalin, (arg$^6$)-leu-enkephalin, alpha-neo-endorphin, beta-neo-endorphin, dynorphin, PH-8P (dynorphin (1—8)), and beta-endorphin.

4. Use of an endogenous endorphin according to claim 1, characterized in that the endogenous endorphin is leucine-enkephalin.

5. Use of an endogenous endorphin according to claim 1, characterized in that the endogenous endorphin is methionine-enkephalin.

6. Use of an endogenous endorphin according to claim 4 or 5, characterized in that the enkephalin is administered in the range from 0.001 to 30 mg/kg.


**Patentansprüche**

1. Verwendung eines endogenen Endorphins zur Herstellung eines Arzneimittels zum Hemmen von Tumorwachstum und/oder Stimulieren des natürlichen Immunsystems, welches dem Tumorwachstum Widerstand leistet.

2. Verwendung eines endogenen Endorphins nach Anspruch 1, dadurch gekennzeichnet, daß das endogene Endorphin ausgewählt ist aus beta-Endorphin (61—91) und Peptiden, deren Vorstufe beta-Endorphin (61—91) ist.

3. Verwendung eines endogenen Endorphins nach Anspruch 1, dadurch gekennzeichnet, daß das endogene Endorphin ausgewählt ist aus Met-Enkephalin, Leu-Enkephalin, (Arg$^6$)-Leu-Enkephalin, alpha-Neo-Endorphin, beta-Neo-Endorphin, Dynorphin, PH-8P (Dynorphin (1—8)) und beta-Endorphin.

4. Verwendung eines endogenen Endorphins nach Anspruch 1, dadurch gekennzeichnet, daß das endogene Endorphin Leucyn-Enkephalin ist.

5. Verwendung eines endogenen Endorphins nach Anspruch 1, dadurch gekennzeichnet, daß das endogene Endorphin Methionin-Enkephalin ist.

6. Verwendung eines endogenen Endorphins nach den Ansprüchen 4 oder 5, dadurch gekennzeichnet, daß das Enkephalin in dem Bereich von 0,001 bis 30 mg/kg verabreicht wird.

# 0 076 676

**Revendications**

1. Utilisation d'une endorphine endogène pour la fabrication d'un médicament pour inhiber la croissance tumorale et/ou pour stimuler le système immunitaire naturel s'opposant à la croissance tumorale.

2. Utilisation d'une endorphine endogène selon la revendication 1, caractérisée en ce que l'endorphine endogène est choisie parmi la bêta-endorphine (61—91) et les peptides dont la bêta-endorphine (61—91) est le précurseur.

3. Utilisation d'une endorphine endogène selon la revendication 1, caractérisée en ce que l'endorphine endogène est choisie parmi la met-enképhaline, la leu-enképhaline, l'(arg$^6$)-leu-enképhaline, l'alpha-néo-endorphine, la bêta-néo-endorphine, la dynorphine, la PH-8P (dynorphine (1—8)) et la bêta-endorphine.

4. Utilisation d'une endorphine endogène selon la revendication 1, caractérisée en ce que l'endorphine endogène est la leucine-enképhaline.

5. Utilisation d'une endorphine endogène selon la revendication 1, caractérisée en ce que l'endorphine endogène est la la méthionine-enképhaline.

6. Utilisation d'une endorphine endogène selon la revendication 4 ou 5, caractérisée en ce que l'enképhaline est administrée à raison de 0,001 à 30 mg/kg.

7